# EUROPEAN PATENT SPECIFICATION

(11) **EP 2 532 307 B1**
(45) Date of publication and mention of the grant of the patent: **22.07.2015**
(21) Application number: 12170524.8
(22) Date of filing: 01.06.2012
(51) Int. Cl.: A61B 8/00, A61B 8/14, A61B 8/08

(54) **Apparatus for user interactions during ultrasound imaging**
Vorrichtung für Benutzerinteraktionen bei der Ultraschallabbildung
Appareil pour interactions de l'utilisateur pendant une imagerie ultrasonore

(30) Priority: 10.06.2011 CN 201110156991; 10.11.2011 US 201161558423 P; 14.03.2012 US 201213420589
(43) Date of publication of application: 12.12.2012
(73) Proprietor: Chison Medical Imaging Co., Ltd., New District Wuxi Jiangsu 214028 (CN)
(72) Inventor: Mo, Ruoli, 214028 Wuxi (CN); Gong, Dongliang, 214028 Wuxi (CN); Zhao, Minghang, 214028 Wuxi (CN); Zhao, Danhua, 214028 Wuxi (CN)
(74) Representative: Finnegan Europe LLP

(56) References cited:
- EP-A1- 2 196 150
- US-A1- 2008 119 731
- US-A1- 2010 004 539
- US-A1- 2010 094 132

## Description

### FIELD OF TECHNOLOGY

The disclosure of the present application relates to, but not limited to, ultrasound imaging and user input processing for ultrasound imaging.

### BACKGROUND

The conventional medical ultrasound imaging systems configured with touch screen displays have a number of problems and limitations. In a conventional ultrasound imaging system, a screen is typically divided into two areas: an image area and a control area. The majority of the screen display is allocated for the image area, leaving a small region for the control area that typically displays a graphical representation of function buttons. The function buttons displayed within the control area are typically configured to be associated with the global processing of the entire ultrasound image. Some of function buttons presented in the conventional ultrasound imaging systems are confusing and non-intuitive to a novice user.

EP 2 196 150 A1 discloses a hand-held ultrasound system including a touch screen on which the ultrasound images and a touch screen menu is shown, the touch screen menu being displayed in a region in proximity to a location touched by a finger of a user when the hand-held system is held by a single hand.

US 2010/0004539 A1 discloses an ultrasound imaging apparatus according to the preamble of claim 1.

### BRIEF DESCRIPTION OF THE DRAWINGS

The embodiments disclosed in the presented application are illustrated by way of example and not limitation in the figures of the accompanying drawings in which like references indicate similar elements.

**Figure 1** shows an ultrasound imaging system according to one embodiment.

**Figure 2** shows a user interface for an ultrasound imaging system according to one embodiment.

**Figure 3** shows a portion of an ultrasound imaging system configured to process touch input according to one embodiment.

**Figure 4** illustrates an example of localized image processing according to one embodiment.

**Figure 5** shows a method to process touch input according to one embodiment.

### SUMMARY OF THE DESCRIPTION

According to the invention, an ultrasound imaging apparatus includes: an ultrasound transducer; a beamformer coupled with the ultrasound transducer to transmit and receive acoustic/ultrasound beams; an image processor coupled with the beamformer to generate an ultrasound image; a touch screen display coupled with the image processor to display the ultrasound image; and a touch processor coupled with the touch screen display to process touch input received from the touch screen display.

In one aspect, the touch processor is configured to receive the touch input on a portion of the touch screen display, on which portion the ultrasound image is currently being displayed; and the touch processor is configured to identify a command corresponding to the touch input and cause the execution of the command as the response to the touch input. Thus, the touch input is interpreted as a request to execute the command.

In one embodiment, the touch processor includes one of: an application-specific integrated circuit and a field programmable circuit; and the image processor includes one of: an application-specific integrated circuit and a field programmable circuit. In one embodiment, the touch processor and image processor are implemented using distinctly separate hardware. Alternatively, the touch processor and image processor may share a general purpose microprocessor, controlled via separate firmware or software.

According to the invention, the touch processor is configured to communicate the identified command to the image processor, which executes the command. The command may cause the image processor to transform the ultrasound image, or to adjust an imaging processing operation for the generation of the ultrasound image.

According to the invention, the touch processor is coupled with the image processor to request image information derived from the ultrasound image; and the touch processor is configured to use both the image information and the touch input to identify the command.

In one embodiment, the touch input identifies a region within the ultrasound image and instructs the image processor to perform an image analysis within the region to provide the image information.

In one embodiment, the touch input identifies a region within the ultrasound image and requests the image processor to execute the command within the region.

In one embodiment, when the command is executed, a graphical representation of a user interface element is presented over the ultrasound image to receive further touch input. The graphical representation of a user interface element is translucent in one embodiment to partially reveal the ultrasound image on which the graphical representation of a user interface element is overlaid.

The disclosure includes methods and apparatuses which perform these methods, including data processing systems which perform these methods, and computer readable media containing instructions which when executed on data processing systems cause the systems to perform these methods.

Other features will be apparent from the accompanying drawings and from the detailed description which follows.

### DETAILED DESCRIPTION

The following description and drawings are illustrative and are not to be construed as limiting. Numerous specific details are described to provide a thorough understanding. However, in certain instances, well known or conventional details are not described in order to avoid obscuring the description.

References to one or an embodiment in the present disclosure are not necessarily references to the same embodiment; and, such references mean at least one. The present disclosure includes all combinations of various features discussed in connection with different references to one or an embodiment, except certain combinations are excluded by ways of explicit discussion or obvious incompatibility.

In one embodiment, an ultrasound imaging system is provided with a touch screen based user interface, which is configured to allow users to provide input based at least in part on touching a portion of a touch screen display, on which portion an ultrasound image is currently being displayed while the touching input is received. Allowing a user to provide touch input on the portion of the touch screen display where the ultrasound image is currently being displayed provides a large touch surface for input operations and allow the system to interpret touch input in a context sensitive manner to provide improved user experience.

In one embodiment, the touch screen based user interface allows the user to select, via touching the displayed image, a sub-region to cause the imaging system to perform, change, modify and/or adjust image processing tasks on the displayed image based on the region selected by the user.

In one embodiment, instead of utilizing only the user touch input to select a command that corresponding to the touch input, the touch screen based user interface identifies a command and/or parameters for the execution of the command, based on not only the touch input but also the underlying image that is being touched. In one embodiment, the underlying image is analyzed for the touch screen based user interface; and the analysis result is combined, by the touch screen based user interface, with the touch input to generate intelligently a command appropriate for the processing of the underlying image and corresponding to the touch input.

In one embodiment, the underlying image is a live ultrasound image, which updates periodically according to the most recent signals from the ultrasound transducer. In one embodiment, the underlying image is a frozen ultrasound image, which is based on the signals from the ultrasound transducer during a particular time period and not further updated according to recent signals from the ultrasound transducer.

Systems and methods of a touch screen based user interface in medical ultrasound imaging systems according to some embodiments are described in further details below.

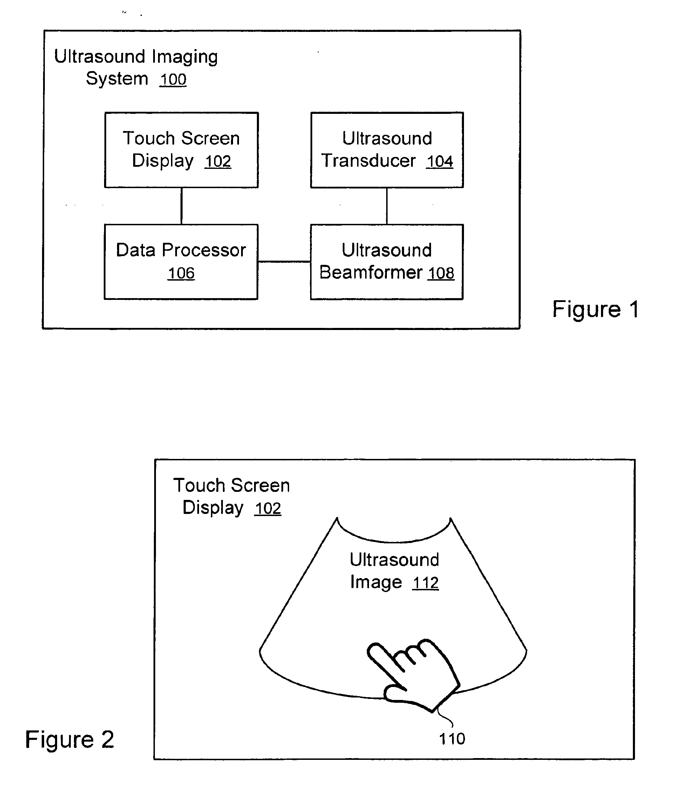

**Figure 1** shows an ultrasound imaging system according to one embodiment. In **Figure 1****,** the ultrasound imaging system 100 includes an ultrasound transducer 104, a ultrasound beamformer 108 coupled with the ultrasound transducer 104, a touch screen display 102 and a data processor 106 coupled with the beamformer 108 to generate ultrasound image and coupled with the touch screen display 102 to display the ultrasound image and receive user touch input.

In one embodiment, the data processor 106 is coupled with the ultrasound beamformer 108 to transmit electrical pulses to the ultrasound transducer 104 and receive echo signals from the ultrasound transducer 104.

**Figure 2** shows a user interface for an ultrasound imaging system according to one embodiment. In **Figure 2****,** the ultrasound image 112 generated based on the signals from the ultrasound transducer 104 is displayed in at least portion of the touch screen 102.

In one embodiment, while the ultrasound image 112 is displayed on a portion of the touch screen 102, one or more user interface elements can be displayed concurrently on a different portion of the touch screen 102. Examples of such one or more user interfaces elements include icon image representations of function buttons, which can be selected via touch to activate the execution of predetermined image functions or to invoke other user interfaces to replace the currently displayed user interface elements and/or the ultrasound image 112.

In one embodiment, the ultrasound image 112 is displayed on a portion of the touch screen 102 without concurrently displaying other conventional graphical user interface elements, such as icon buttons, menu buttons, text fields for receiving text input, etc. Some conventional graphical user interface elements may be overlaid on the ultrasound image 112 in response to the user touch input on the ultrasound image 112.

In one embodiment, the user interface is configured to allow the user to touch the ultrasound image 112 displayed on the touch screen 102 to provide the touch input. The touch input is received while the ultrasound image 112 is displayed on the corresponding portion of the touch screen 102 being touched, without the display of a conventional graphical user interface element on the corresponding portion of the touch screen 102.

In one embodiment, the touch input on the ultrasound image 112 is interpreted to generate an image processing command to process the ultrasound image 112 and provide an updated display of the ultrasound image 112.

In one embodiment, different touch inputs are interpreted by the user interface as different touch gestures; and different touch gestures are pre-associated with different, pre-defined user interface functions (e.g., open a predefined user interface, open a pre-identified document or form) and/or image processing functions (e.g., zooming in or zooming out, edge enhancing the image, adjusting the brightness or contrast of the image).

In one embodiment, a touch input represents a user request to improve the image; and the image is analyzed to select an image processing function to improve the image. In one embodiment, the improvement is based on the analysis of a local region touched by the user; and the image processing function may be applied to the same local region, or the entire ultrasound image. In one embodiment, the improvement is applied on a local region touched by the user, based on the image analysis of the location region or the entire ultrasound image.

In one embodiment, the touch gestures are mapped to predefined user interface operations without relying upon the image properties of the ultrasound image 112 being touched. Thus, the same touch gesture applied on different ultrasound images 112 causes the system to apply the same user interface function or image processing function that is pre-associated with the touch gesture.

In one embodiment, a touch gesture includes a sequence of touching operations that identify one or more locations or regions in the ultrasound image 112 and a desired operation based on the identified one or more locations or regions. In one embodiment, the identified one or more locations or regions are used as one or more parameters for the desired operation in processing the ultrasound image 112.

In one embodiment, the ultrasound image 112 as displayed is considered a graphical user interface element, which may have a set of user interface/image processing functions pre-associated with the graphical user interface element, regardless of the content of the ultrasound image 112.

In one embodiment, the ultrasound image 112 as displayed is considered a graphical user interface element, which may have a set of user interface/image processing functions associated with the graphical user interface element based on the certain properties of the ultrasound image 112. The properties of the ultrasound image 112 may be based on a statistical analysis of the ultrasound image 112 (e.g., brightness, contrast) or based on a pattern recognition analysis of ultrasound image 112 (e.g., predefined tissue features captured in the ultrasound image 112). Different portions of the ultrasound image 112 that have different image characteristics and/or captured features or image patterns may be assigned dynamically with different graphical user interface functions.

In one embodiment, the properties of the ultrasound image 112 are determined in response to touch input and used to determine the graphical user interface functions to be invoked in response to the touch input. The properties are determined based on the entire ultrasound image 112 as displayed in one embodiment or alternatively, based on a portion of the ultrasound image 112 as identified via the touch gesture.

For example, in one embodiment, when a portion of the ultrasound image 112 as identified via the touch gesture has a contrast level below a threshold, the touch gesture is interpreted as a request to improve the contrast level; and when the contrast level is with a predetermined range, the touch gesture is interpreted as a request to perform edge enhancement on the ultrasound image 112, if there are detected edge in the area touched by the hand 110 of the user (or a stylus).

In one embodiment, the ultrasound image 112 is analyzed to generate a scale of operation; and the touch gesture is interpreted based on the scale of operation to generate parameters for an image processing operation that is performed as a response to the touch gesture.

When the touch input/gesture is interpreted based on the context provided by the image properties and/or features captured in the ultrasound image 112, the user interface can process the touch input more intelligently.

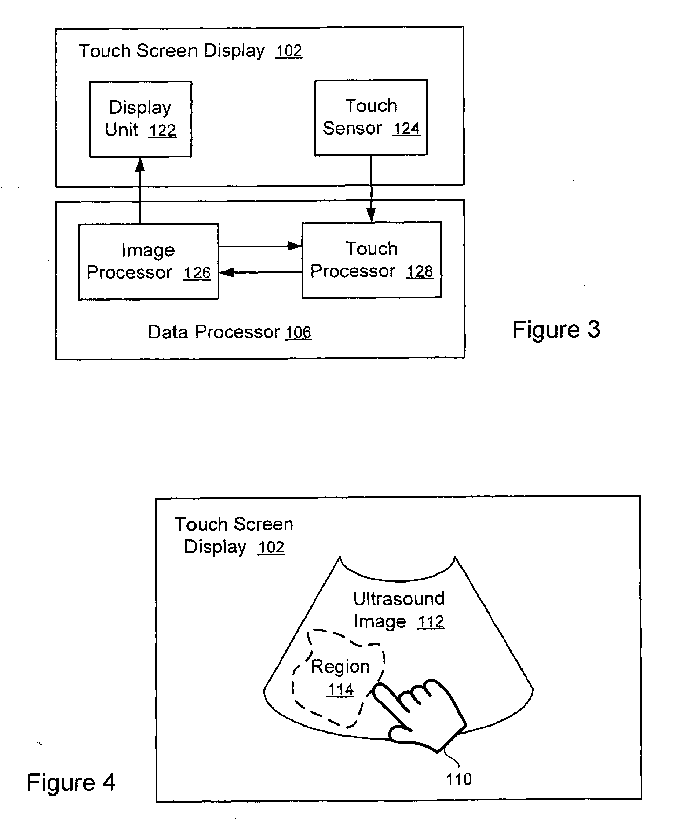

**Figure 3** shows a portion of an ultrasound imaging system configured to process touch input according to one embodiment. In **Figure 3****,** the touch screen display 102 includes a display unit 122 configured to display images and a touch sensor 124 configured to detect touch input on the display unit 122.

In one embodiment, the touch sensor 124 is configured for the measurement of the locations over the display unit touched by one or more human fingers. In one embodiment, the touch sensor 124 is also configured for the measurement of the pressure of the touch. Various touch sensing technologies, currently known in the field and developed in the future, can be used in various embodiments of the touch sensor 124. The disclosure is not limited to a particular type of touch sensing technique.

In **Figure 3****,** the data processor 106 includes an image processor 126 configured to generate images for display on the display unit 122 (e.g., based on the signal input from the ultrasound transducer 104). The data processor 106 further includes a touch processor 128 configured to process the touch input generated by the touch sensor 124. The touch sensor 124 is configured to generate processing commands in response to the touch input received from the touch sensor 124.

In one embodiment, the touch processor 128 is configured to generate commands based on not only the touch input received from the touch sensor 124, but also the image information received from the image processor 126.

In one embodiment, the ultrasound image 112 displayed on the display unit 122 is analyzed to provide an image context; and the touch sensor 124 is configured to interpret the touch input based on the image context to identify, select, or generate a context sensitive processing command for the image processor.

In one embodiment, the image context is determined based on a portion of the touch input. For example, a touch input may specify one or more locations or a region; and the touch processor 128 is configured to request the image processor to obtain the image context based on the one or more locations or a region in the ultrasound image 112; and the image context is further used to interpret the remaining portion of the touch input to transform the ultrasound image 112.

Examples of the image context include the brightness level of the ultrasound image112, the contrast level of the ultrasound image112, a pattern recognized as being corresponding to a feature (e.g., a bone, an organ, a tumor), the location and/or size of the recognized feature, etc.

For example, in one embodiment, a touch input at a location corresponding to a recognized feature may be interpreted as a request to adjust the zoom level and the center point to present the recognized feature using the available display area on the display unit 122, when the recognized feature is currently displayed in a small area of the display unit.

For example, in one embodiment, when a touch input is at an ultrasound image having a contrast level lower than a predetermine threshold but not at a location on a recognized feature, the touch input is interpreted as a request to adjust the contrast level of the ultrasound image 112.

Thus, in one embodiment, the touch processor 128 takes the user inputs originated from the touch sensor 124 of the touch screen display 102 and image information derived by the image processor from the ultrasound image 112 currently being displayed to on the display unit 122 of the touch screen display 102 to intelligently generate commands or instructions to operate the image processor 126 and/or the ultrasound imaging system 100.

In one embodiment, the user inputs originated from the touch sensor 124 and combined with the image information for the determination of the commands or instructions include the inputs from the area currently used to display the ultrasound image 112 and/or the inputs from the area configured to display graphical user interface elements, such as icon buttons, menu buttons, sliders, etc.

In one embodiment, in response to the user touch input, the data processor 126 is configured to generate one or more conventional graphical user interface elements, such as buttons, sliders, text input boxes, etc., which are temporarily displayed over the ultrasound image 112 to further collect user inputs. In one embodiment, the displayed conventional graphical user interface elements overlaid on the ultrasound image 112 is translucent, allowing the user to partially see ultrasound image 112 through the displayed conventional graphical user interface elements. The translucent graphical user interface elements provide hints to assist the user in providing input.

For example, when there are ambiguities regarding to the intent of the user, a plurality of choices can be presented to allow the user to select one from a plurality of possible processing options.

For example, when there are multiple processing options of similar priorities, the options can be displayed for an explicit selection by the user.

For example, the graphical user interface elements may be presented in one embodiment to receive user specified parameters for an operation selected based on the image context and the previous touch input.

Various types of image information can be derived from either a local region of the ultrasound image 112 touched by the user or the entire ultrasound image 112. The derived image information, which is typically different from the ultrasound image 112 itself, can be used by the touch processor 128 to deduce optimal commands for controlling the image processor 126 and/or the ultrasound imaging system 100. In one embodiment, the deduced commands may cause the image processor 126 to perform a local image processing operation within the local region identified via user touching or a global image processing operation for the entire ultrasound image 112.

Examples of the image processing operations include imaging parameter adjustment, zooming, translation, measurement, editing, deletion, copying, and combinations of such image processing operations. The imaging parameter adjustment can be applied to a local region touched by the user or the global ultrasound image 112. Examples of the imaging parameters that may be adjusted via such touch inputs include spatial resolution, temporal resolution, contrast, dynamic range, focal point, display depth, gain, time gain control (TGC), pulse repetition frequency (PRF), etc.

In one embodiment, the touch processor 128 is configured to determine the touch direction, speed, position, pressure, order of multiple touch operations, and combinations of multiple touch operations to determine an image processing operation intended by the user.

Examples of image information and properties that can be used as the image context for the determination of the image processing operation intended by the user include statistics data, such as mean brightness, noise level, different tissue texture, tissue motion, boundary, etc.

In one embodiment, the touch processor 128 is configured to detect one or more input signals from the touch sensor 124 of the touch screen display 102; and in response, the touch processor 128 analyzes the input signals to recognize one or more predefined touch gestures. The touch processor 128 optionally instructs the image processor 126 to process and analyze the currently displayed ultrasound image 112 to obtain image analysis results. Based on the image analysis results and the recognized touch gestures, the touch processor 128 is configured to generate one or more commands instructing the image processor 126 to transform the ultrasound image 112 or adjust the mode or parameters to generate the ultrasound image 112.

In one embodiment, the touch processor 128 provides the recognized touch gesture to the image processor 126; and the image processor 126 is configured to use the image analysis results and the recognized touch gesture to determine the one or more commands for transforming the ultrasound image 112, or adjusting the mode or parameters to generate the ultrasound image 112.

Examples of image analyses to obtain image context for the determination of the one or more commands include, but not limited to, filtering, statistical analyses, feature exaction, edge detection, and pattern recognition. For instance, local image mean brightness can be computed for image based dynamic gain control (DGC) adjustment.

Examples of the determined command include, but not limited to, image optimization, edge enhancement, restoration, segmentation, and imaging parameter adjustment.

In one embodiment, the image analysis is performed for a local region within the ultrasound image 112 to obtain the image context for the determination of the one or more commands. For example, a user may draw an arbitrary shape via touching, using a hand 110 (or a stylus), to define a region of interest (ROI) 114 as illustrated in **Figure 4****.** After the region 114 is defined via touching, the user may further tap a point inside the region 114 to request the region 114 to be processed.

In one embodiment, the image processor 126 analyzes the region 114 of the ultrasound image 112 but not the region outside the region 114 to determine a local image context, based on which a processing command is determined for the tap at the point side the region 114.

In one embodiment, the image processor 126 analyzes the ultrasound image 114, including both the region 114 and the region outside the region 114, to determine a global image context, based on which a processing command is determined for the tap at the point side the region 114.

In one embodiment, the processing command, determined based on the local image content or the global image context, is applied to transform the region 114 within the ultrasound image 112, but not the region outside the region 114. Alternatively, the processing command can be applied globally to the entire ultrasound image 112.

In one embodiment, the region of interest (ROI) 114 has an arbitrary shape, determined according to the path of touch made via the hand 110 of the user. In one embodiment, the path of touch is converted into a regular shape, such as a box, an oval, a circle, etc., to simplify processing.

In one embodiment, the image analysis for the image context and/or the processing command to transform is applied to the live ultrasound images that are periodically updated according to the input from the ultrasound transducer 104. Alternatively, the image analysis and/or the processing command can be applied to a frozen image that is based on a particular set of input obtained from the ultrasound transducer 104 within a particular time period, where the input from the ultrasound transducer 104 is not updated with the passage of time.

In one embodiment, a dynamic ROI 114 is generated according to the location and motion of a user finger or a pointing device, such as a stylus. Image analysis performed within the ROI 114 to obtain the image context may be the operation of filtering, statistical analyses, feature exaction, edge detection, and pattern recognition, etc. to obtain local image context. Such local image analysis can be applied to the live ultrasound image 112 or a frozen ultrasound image 112.

In one embodiment, after a dynamic ROI 114 is identified, an adaptive image optimization process is applied to the local image inside the ROI 114 as a response to a tap on a point inside the region 114. For instance, in response to the tap on the point inside the region 114, edge enhancement is applied if the ROI 114 contains boundaries; or a smoothing filter is applied if the ROI 114 has a speckle noise level above a threshold. Thus, the results of the boundary detection and speckle noise level evaluation performed on the ROI 114 provide the image context for the selection of the edge enhancement operation and/or the smoothing filter operation, as a response to the touch input on the ROI 114 from the user.

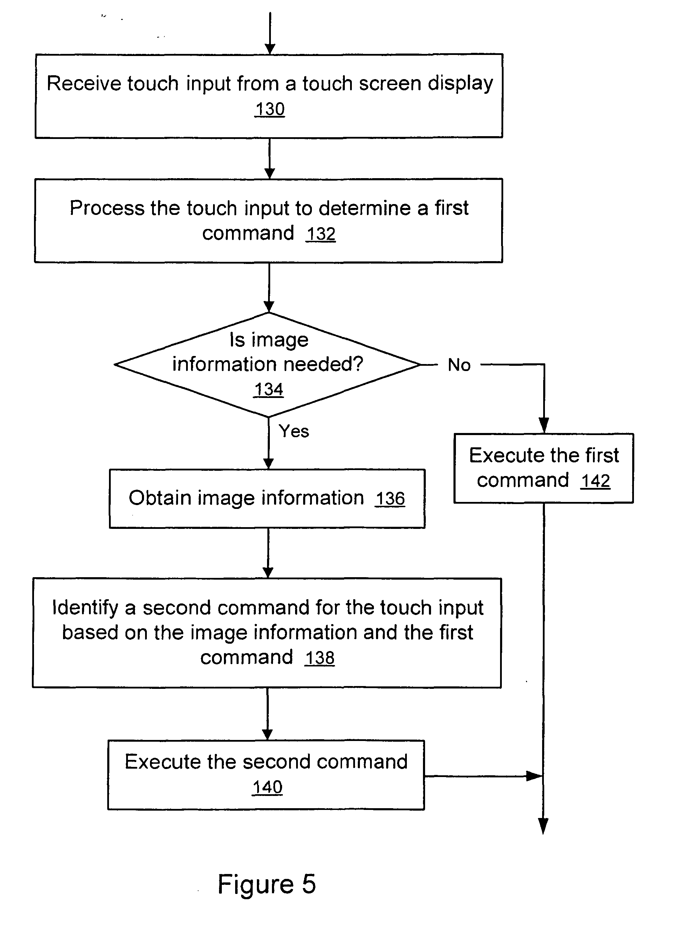

**Figure 5** shows a method to process touch input according to one embodiment. In **Figure 5****,** after a touch processor 128 receives 130 touch input from a touch sensor 124 of a touch screen display 102, the touch processor 128 processes 132 the touch input to determine a first command.

In one embodiment, at least a portion of the touch input is received from an area of the display unit 122 of the touch screen display 102, on which area the ultrasound image 112 is displayed.

In one embodiment, at least a portion of the touch input is received from an area that is outside the ultrasound image 112 and that displays graphical representation of user interface elements that are separate from the ultrasound image 112.

In one embodiment, at least a portion of the touch input is received from a graphical representation of one or more user interface elements overlaid in a partially transparent manner over the ultrasound image 112.

In one embodiment, at least a portion of the touch input identifies a region 114 of interest within the ultrasound image 112.

In **Figure 5****,** the touch processor 128 determines 134 whether image information (e.g., information about the ultrasound image 112 displayed on the touch screen display 102) is needed to further process the touch input.

In one embodiment, the first command corresponding to a plurality of command candidates; and the image information is used to select one or more commands from the plurality of command candidates.

In one embodiment, the first command requires one or more parameters; and the image information is used to determine the one or more parameters based on the touch input.

In one embodiment, the first command has one or more options; and the image information is used to select an option.

In one embodiment, the first command is applied to the image information to generate a second command.

In **Figure 5****,** if the touch processor 128 determines 134 that the image information is needed, the touch processor 128 obtains the image information from an image processor 126, which controls the display of images on the touch screen display 102; otherwise, the ultrasound imaging system 100 executes 142 the first command.

In one embodiment, the image information is generated based on an analysis of the image displayed on the touch screen, such as statistical analysis, filtering, feature exaction, edge detection, and/or pattern recognition, etc.

In one embodiment, the image information is different from the ultrasound image displayed on the touch screen display 102.

In **Figure 5****,** the touch processor 128 identifies 138 a second command for the touch input based on the image information and the first command; and the ultrasound imaging system 100 executes the second command.

For example, the second command may instruct the image processor 126 to adjust the image processing operations for the display of the image on the touch screen, such as image optimization, enhancement, restoration, segmentation, and imaging parameter adjustment, etc.

In one embodiment, the image processor 126 and the touch processor 128 are implemented using separate hardware. For example, image processor 126 and the touch processor 128 can be implemented using application-specific integrated circuit (ASIC) or field-programmable gate array (FPGA). Alternatively, the image processor 126 and the touch processor 128 can be implemented using general purpose microprocessor controlled by firmware and/or software. In one embodiment, the image processor 126 and the touch processor 128 are configured to share one or more microprocessors.

Thus, at least one embodiment of the disclosure provides an ultrasound imaging system configured with a touch screen based user interface, which addresses at least one of the limitations and/or problems in the conventional medical ultrasound imaging system equipped with touch screens.

An ultrasound imaging system according to one embodiment of the disclosure includes an ultrasound transducer 104, a touch screen display 102, a beamformer 108, a data processing unit (e.g., data processor 106) and an electronic circuit interconnecting the components.

In one embodiment, the data processing unit is configured to perform signal processing, image processing, and input/output handling.

In one embodiment, the input/output handling includes receiving touch input from the image area displayed on the touch screen display and using image information derived from the ultrasound image 112 currently being displayed on the touch screen display 102 to generate intelligently proper commands or instructions corresponding to the touch input.

In one embodiment, in response to the touch input received from the image area, the touch input is processed and analyzed to determine the intended instructions by the user. According to the determined instructions by the user, an image analysis is performed to derive image information from either a local region of the image presented on the image area of the touch screen display 102, or the entire ultrasound image 112. Based on the derived image information, one or more commands are then generated to perform imaging tasks for the processing the live or frozen ultrasound image 112.

In one embodiment, the commands determined in accordance with the touch input and the derived image information are executed for local image processing within a location region 114 identified by the user touch input. Alternatively or in combination, the commands may be executed for global image processing of the entire ultrasound image 112.

In one embodiment, a local region is predefined or preselected. In one embodiment, a local region 114 is defined or selected by the user via touch input. In one embodiment, the local region is where the user is touching (e.g., via simultaneously touching multiple points using multiple fingers, via sequentially touching a set of discrete points using one finger, or via sliding one or more fingers on the touch screen); and the image in the local region is a portion of the ultrasound image 112 displayed on the touch screen display 102.

Limiting the processing of the image to a local region reduces the processing load on the data processing unit and can improve the response time. Thus, the result of the image processing can be provided in real time as the user providing input via touch input. For example, when the global image has 500 x 600 pixels and the local image has 20 x 30 pixels, the time used for the processing of the local image can be much shorter than that for the global image.

In one embodiment, the image processing operations associated with the determined instructions include imaging parameter adjustment, zooming, translation, measurement, editing, deletion, and copying. The imaging parameter adjustment can be either local or global.

In one embodiment, a local image processing operation is applied to the portion of image that is displayed within a predefined region (e.g., the image area of the touch screen, or a portion of the image area of the touch screen); and a global image processing operation is applied to the entire ultrasound image which may have a portion that is outside the predefined region.

In one embodiment, the imaging parameters that can be adjusted via the determined instructions include spatial resolution, temporal resolution, contrast, dynamic range, focal point, display depth, gain, time gain control (TGC), pulse repetition frequency (PRF), etc.

In one embodiment, the data processing unit is configured to detect direction, speed, position, pressure, and order of user operations, and combinations of multiple user touch inputs. The detected direction, speed, position, pressure, and order of user operations, and combinations of multiple user touch inputs are used to determine the intended instructions of the user.

In one embodiment, the image information and properties that can be displayed in accordance with the intended instructions of the user include statistics such as mean brightness, noise level, different tissue texture, tissue motion, boundary, etc.

In one embodiment, the data processing unit is configured to detect input signal from the touch screen, analyze the input signal to determine the intended instruction of the user, process and analyze the displayed ultrasound image in accordance with the determined instruction of the user, and provide an output on the touch screen display based on the input instruction and the result of the image analysis.

In one embodiment, the touch input of the user can be generated not only from the control area of the touch screen, but also the image area of the touch screen.

In one embodiment, the properties of ultrasound image are processed and analyzed, and then combined with the user input instructions from the touch screen to generate proper commands, such as an optimal command that will be carried out to perform certain tasks, such as image enhancement. For example, the local image brightness is determined and used in one embodiment to generate gain adjustment commands for processing a local region of the ultrasound image in accordance with a touch input received in the image region of the touch screen display.

In one embodiment, local image processing and optimization are performed in response to the determined user instruction and based on local image properties.

In one embodiment, the touch screen operations are reconfigurable. The user can change or redefine some or all of predefined touch screen operations. For instance, a left-to-right move can be redefined as brightness increase instead of the default meaning: forward replay.

In one embodiment, after a touch input requesting reconfiguration is received from the touch screen display 102, the touch processor 128 analyzes and recognizes touch inputs that are provided by the user as the training inputs. The training inputs are used to determine parameters for recognize subsequent touch inputs that are intended to signal the user request corresponding to the instruction or command that is being reconfigured. Thus, the touch recognition for the instruction or command, corresponding to a touch gesture is reconfigured based on the training results.

In one embodiment, for instance, in the default setting, pressing a point on an image with a finger tip for a certain amount of time like 2 seconds brings transmitting focus to the nearest location of the finger tip; the same operation can be reconfigured to perform a different task like drawing a box of a predetermined size corresponding to a region 114 of interest.

In the above discussion, examples of touch screen input processing are presented in connection with ultrasound imaging systems. However, the touch screen image processing techniques can also be applied to other types of imaging systems and systems configured for displaying images, such as computed axial tomography (CAT or CT) systems, magnetic resonance imaging (MRI) systems, photoacoustic imaging systems, etc.

In the foregoing specification, the disclosure has been described with reference to specific exemplary embodiments thereof. The specification and drawings are, accordingly, to be regarded in an illustrative sense rather than a restrictive sense.

## Claims

1. An ultrasound imaging apparatus, comprising:
an ultrasound transducer (104);
a beamformer (108) coupled with the ultrasound transducer (104) to transmit and receive beams;
an image processor (126) coupled with the beamformer (108) to generate an ultrasound image;
a touch screen display (102) coupled with the image processor (126) to display the ultrasound image; and
a touch processor (128) coupled with the touch screen display (102) to receive touch input on a portion of the touch screen display (102) on which portion the ultrasound image is currently being displayed, the touch processor (128) configured to identify a command corresponding to the touch input and cause an execution of the command in response to the touch input,
**characterized in that**
the touch processor (128) is coupled with the image processor (126) to request image information derived from the ultrasound image; and the touch processor (128) is configured to use the image information and the touch input to identify the command wherein the touch input is for identifying a region within the ultrasound image and instructing the image processor to perform an image analysis within the region to provide the image information.

2. The ultrasound imaging apparatus of claim 1, wherein the touch processor (128) includes one of: an application-specific integrated circuit and a field programmable circuit.

3. The ultrasound imaging apparatus of claim 2, wherein the image processor (126) includes one of: an application-specific integrated circuit and a field programmable circuit.

4. The ultrasound imaging apparatus according to any of claims 1-3, wherein the touch processor (128) is configured to communicate the command to the image processor (126), which executes the command.

5. The ultrasound imaging apparatus of claim 4, wherein the command is for causing the image processor (126) to transform the ultrasound image.

6. The ultrasound imaging apparatus of claim 4, wherein the command is for causing the image processor (126) to adjust an imaging processing operation for the ultrasound image.

7. The ultrasound imaging apparatus of claim 1, wherein the touch input is for identifying a region within the ultrasound image and requesting the image processor (126) to execute the command within the region.

8. The ultrasound imaging apparatus of claim 1, wherein when the command is executed, a graphical representation of a user interface element is presentable over the ultrasound image to receive further touch input.

## Patentansprüche

1. Vorrichtung zur Bilddarstellung mittels Ultraschall, umfassend:
einen Ultraschallmesswandler (104);
einen Beamformer (108), der mit dem Ultraschallmesswandler (104) gekoppelt ist, um Strahlen zu senden und zu empfangen;
einen Bildprozessor (126), der mit dem Beamformer (108) gekoppelt ist, um ein Ultraschallbild zu erzeugen;
eine Berührungsbildschirmanzeige (102), die mit dem Bildprozessor (126) gekoppelt ist, um das Ultraschallbild anzuzeigen; und
einen Berührungsprozessor (128), der mit der Berührungsbildschirmanzeige (102) gekoppelt ist, um Berührungseingaben auf einem Abschnitt der Berührungsbildschirmanzeige (102) zu empfangen, wobei das Ultraschallbild auf diesem Abschnitt dem gerade angezeigt wird, wobei der Berührungsprozessor (128) so konfiguriert ist, dass er einen Befehl identifiziert, der der Berührungseingabe entspricht, und die Ausführung des Befehls als Reaktion auf die Berührungseingabe bewirkt;
**dadurch gekennzeichnet, dass**
der Berührungsprozessor (128) mit dem Bildprozessor (126) gekoppelt ist, um Bildinformationen anzufordern, die von dem Ultraschallbild abgeleitet werde; und wobei der Berührungsprozessor (128) so konfiguriert ist, dass er die Bildinformationen und die Berührungseingabe verwendet, um den Befehl zu identifizieren, wobei die Berührungseingabe dazu dient, einen Bereich in dem Ultraschallbild zu identifizieren und den Bildprozessor anzuweisen, eine Bildanalyse in dem Bereich auszuführen, um Bildinformationen bereitzustellen.

2. Vorrichtung zur Bilddarstellung mittels Ultraschall, wobei der Berührungsprozessor (128) eines der folgenden aufweist: einen anwendungsspezifischen integrierten Schaltkreis oder einen Universalschaltkreis.

3. Vorrichtung zur Bilddarstellung mittels Ultraschall nach Anspruch 2, wobei der Bildprozessor eines der folgenden aufweist: einen anwendungsspezifischen integrierten Schaltkreis oder einen Universalschaltkreis.

4. Vorrichtung zur Bilddarstellung mittels Ultraschall nach einem der Ansprüche 1 bis 3, wobei der Berührungsprozessor (128) so konfiguriert ist, dass er den Befehl an den Bildprozessor (126) kommuniziert, der den Befehl ausführt.

5. Vorrichtung zur Bilddarstellung mittels Ultraschall nach Anspruch 4, wobei der Befehl dazu dient, zu bewirken, dass der Bildprozessor (126) das Ultraschallbild umwandelt.

6. Vorrichtung zur Bilddarstellung mittels Ultraschall nach 4, wobei der Befehl dazu dient, zu bewirken, dass der Bildprozessor (126) eine Bildverarbeitungsoperation für das Ultraschallbild anpasst.

7. Vorrichtung zur Bilddarstellung mittels Ultraschall nach Anspruch 1, wobei die Berührungseingabe dazu dient, einen Bereich in dem Ultraschallbild zu identifizieren und den Bildprozessor (126) aufzufordern, den Befehl in dem Bereich auszuführen.

8. Vorrichtung zur Bilddarstellung mittels Ultraschall nach Anspruch 1, wobei, wenn der Befehl ausgeführt wird, eine grafische Darstellung eines Benutzerschnittstellenelements über das Ultraschallbild präsentiert werden kann, um weitere Berührungseingaben zu empfangen.

## Revendications

1. Appareil d'imagerie ultrasonore comprenant :
un transducteur ultrasonore (104) ;
un dispositif de formation de faisceaux (108) raccordé au transducteur ultrasonore (104) pour transmettre et recevoir des faisceaux ;
un processeur d'image (126) raccordé au dispositif de formation de faisceaux (108) pour générer une image ultrasonore ;
un affichage d'écran tactile (102) raccordé au processeur d'image (126) pour afficher l'image ultrasonore ; et
un processeur tactile (128) raccordé à l'affichage d'écran tactile (102) pour recevoir une entrée tactile sur une portion de l'affichage d'écran tactile (102), portion sur laquelle l'image ultrasonore est actuellement en train d'être affichée, le processeur tactile (128) étant configuré pour identifier une commande correspondant à l'entrée tactile et amener une exécution de la commande en réponse à l'entrée tactile,
**caractérisé en ce que**
le processeur tactile (128) est raccordé au processeur d'image (126) pour demander des informations d'image dérivées de l'image ultrasonore ; et le processeur tactile (128) est configuré pour utiliser les informations d'image et l'entrée tactile afin d'identifier la commande, dans lequel l'entrée tactile est destinée à identifier une région au sein de l'image ultrasonore et instruire le processeur d'image de réaliser une analyse d'image au sein de la région pour fournir les informations d'image.

2. Appareil d'imagerie ultrasonore selon la revendication 1, dans lequel le processeur tactile (128) inclut un circuit parmi : un circuit intégré spécifique à une application et un circuit programmable sur place.

3. Appareil d'imagerie ultrasonore selon la revendication 2, dans lequel le processeur d'image (126) inclut un circuit parmi : un circuit intégré spécifique à une application et un circuit programmable sur place.

4. Appareil d'imagerie ultrasonore selon l'une quelconque des revendications 1 à 3, dans lequel le processeur tactile (128) est configuré pour communiquer la commande au processeur d'image (126) qui exécute la commande.

5. Appareil d'imagerie ultrasonore selon la revendication 4, dans lequel la commande est destinée à amener le processeur d'image (126) à transformer l'image ultrasonore.

6. Appareil d'imagerie ultrasonore selon la revendication 4, dans lequel la commande est destinée à amener le processeur d'image (126) à ajuster une opération de traitement d'image pour l'image ultrasonore.

7. Appareil d'imagerie ultrasonore selon la revendication 1, dans lequel l'entrée tactile est destinée à identifier une région au sein de l'image ultrasonore et demander au processeur d'image (126) d'exécuter la commande au sein de la région.

8. Appareil d'imagerie ultrasonore selon la revendication 1, dans lequel, lorsque la commande est exécutée, une représentation graphique d'un élément d'interface utilisateur peut être présenté sur l'image ultrasonore pour recevoir une autre entrée tactile.
